# EUROPEAN PATENT SPECIFICATION

(11) **EP 1 015 019 B1**
(45) Date of publication and mention of the grant of the patent: **27.04.2005**
(21) Application number: 98921004.2
(22) Date of filing: 06.05.1998
(51) Int. Cl.: A61K 38/30, A61P 9/00, A61P 11/00, A61P 25/00

(54) **COMPOSITIONS AND METHODS OF THERAPY FOR IGF-I-RESPONSIVE CONDITIONS**
MITTEL UND THERAPIEVERFAHREN FÜR AUF IGF-I ANSPRECHENDE ERKRANKUNGEN
COMPOSITIONS ET PROCEDES DE THERAPIE DES TROUBLES SUSCEPTIBLES D'ETRE TRAITES PAR IGF-I

(30) Priority: 06.05.1997 IT MI971042
(43) Date of publication of application: 05.07.2000
(73) Proprietor: CHIRON CORPORATION, Emeryville, California 94608-2916 (US)
(72) Inventor: SCHARSCHMIDT, Bruce, F., San Francisco, CA 94127 (US); GORIO, Alfredo, I-20124 Milano (IT); MÜLLER, Eugenio, E., I-20133 Milano (IT)
(74) Representative: Duckworth, Timothy John
(86) International application number: PCT/US1998/009273
(87) International publication number: WO 1998/050062

(56) References cited:
- EP-A- 0 516 901
- DATABASE BIOSIS BIOSCIENCES INFORMATION SERVICE, PHILADELPHIA, PA, US DI GIULIO A M ET AL: "Glycosaminoglycan and insulin-like growth factor treatment of rat neonatal peripheral nerve lesions." XP002075308 & 26TH ANNUAL MEETING OF THE SOCIETY FOR NEUROSCIENCE, WASHINGTON, D.C., USA, NOVEMBER 16-21, 1996. SOCIETY FOR NEUROSCIENCE ABSTRACTS 22 (1-3). 1996. 1960,
- LESMA E ET AL: "Glycosaminoglycans in nerve injury: 1. Low doses of glycosaminoglycans promote neurite formation." JOURNAL OF NEUROSCIENCE RESEARCH, (1996 DEC 1) 46 (5) 565-71, XP002075305
- VERGANI L ET AL: "Effects of low doses of glycosaminoglycans and insulin-like growth factor-I on motor neuron disease in wobbler mouse." NEUROSCIENCE LETTERS, (1997 MAY 30) 228 (1) 41-4, XP002075306 cited in the application
- GORIO A ET AL: "Muscle reinnervation following neonatal nerve crush. Interactive effects of glycosaminoglycans and insulin-like growth factor-I." NEUROSCIENCE, (1998 FEB) 82 (4) 1029-37, XP002075307 cited in the application

## Description

### FIELD OF THE INVENTION

The present invention relates to methods for treatment of mammalian conditions that are responsive to therapy with an insulin-like growth factor or variant thereof.

### BACKGROUND OF THE INVENTION

Insulin-like growth factor I (IGF-I) is a 70-amino-acid polypeptide hormone having insulin-like and mitogenic growth biological activities. This hormone enhances growth and/or survival of cells in a variety of tissues including musculoskeletal systems, liver, kidney, intestines, nervous system tissues, heart, and lung.

Disruption of IGF-I action may contribute to a number of physiological disorders including neurodegenerative disorders such as motor neuron disease, muscular dystrophy, multiple sclerosis, cartilage disorders such as osteoarthritis, bone disease such as osteoporosis, inflammatory disorders such as rheumatoid arthritis, ischemic injuries to organs such as to the heart, brain, or liver, and so forth. Reports of the use of externally supplied IGF-I for therapeutic treatment of such disorders in animal models cite varying results.

For example, a number of studies report on the use of IGF-I as a potential therapeutic agent for treatment of neurodegenerative conditions. See, for example, Kanje *et al*. (1989) *Brain Res.* 486:396-398; Hantai *et al*. (1995) *J. Neurol. Sci.* 129:122-126; Contreras *et al*. (1995) *Pharmac. Exp. Therap*. 274:1443-1499; Di Giulio *et al*. (1996) *Society for Neuroscience* 22:1960; Di Giulio *et al*. (1997) *Society for Neuroscience* 23:894; Hsu *et al*. (1997) *Biochem. Mol. Med.* 60(2):142-148; Gorio *et al*. (1998) *Neuroscience* 82:1029-1037. IGF-I therapy has been indicated in numerous neurological conditions, including ALS, stroke, epilepsy, Parkinson's disease, Alzheimers disease, acute traumatic injury and other disorders associated with trauma, aging, disease, or injury. See, for example U.S. Patent Nos. 5,093,137; 5,652,214; 5,703,045; International Publication Nos. WO 90/1483 and WO 93/02695.

Use of IGF-I therapy for a variety of other conditions has been referred to in a number of publications. See, for example, Schalch *et al*. (1991) in *Modern Concepts of Insulin-Like Growth Factors,* ed. Spencer (Elsevier, New York), pp. 705-714; Clemmons and Underwood (1994) *J. Clin. Endocrinol. Metab.* 79(1):4-6; and Langford *et al*. (1993) *Eur. J. Clin. Invest.* 23(9):503-516) (referring to, *e.g.*, insulin-resistant states and diabetes); and O'Shea *et al*. (1993) *Am. J. Physiol*. 264:F917-F922 (referring to, *e*.*g*., reduced renal function). Also see U.S. Patent Nos. 5,110,604 and 5,427,778 (referring to, *e.g.,* wound healing); 5,126,324 (referring to, *e.g.*, cardiac disorders and growth retardation); 5,368,858 (referring to, *e.g*., defects or lesions in cartilage); 5,543,441/5,550,188 (referring to, *e*.*g*., tissue augmentation); 5,686,425 (referring to, *e.g.*, scar tissue, localized muscular dysfunction, and urinary incontinence); and 5,656,598 (referring to, *e*.*g*., bone growth). Also see International Publication Nos. WO 91/12018 (referring to, *e.g.*, intestinal disorders); WO 92/09301 and WO 92/14480 (referring to, *e.g.,* wound healing); WO 93/08828 (referring to, *e*.*g*., neuronal damage associated with ischemia, hypoxia, or neurodegeneration); WO 94/16722 (referring to, *e.g.*, insulin resistance); WO 96/02565A1 (referring to, *e.g.,* IGF/IGFBP complex for promoting bone formation and for regulating bone remodeling); and European Patent Application No. 560,723 (referring to, *e.g.*, osteoporosis).

Although IGF-I therapy has been referred to for a number of physiological indications, results have sometimes been unpredictable, short-term beneficial effects sometimes don't persist (see, for example, Miller *et al*. (1994) *Kidney International* 46:201-207), and undesirable side effects can result, particularly from administration of high doses and/or long-term administration (see, for example, Jabri *et al*. (1994) *Diabetes* 43:369-374; Wilton (1992) *Acta Paediatr*. 393:137-141). Also, high levels of IGF-I are reportedly correlated with increased risk for prostate cancer (Chan *et al*. (1998) *Science* 278:563-566).

Glycosaminoglycans (GAGs) are complex heteropolysaccharides made up primarily of repeating units of disaccharides, in which one sugar is a hexosamine and the other is a uronic acid. GAGs such as hyaluronic acid and heparin have been used as additives in certain protein-containing pharmaceutical compositions and also have been included as chemoattractants and structural components of certain pharmaceutical compositions, gel formulations, and biodegradable matrices (see, for example, U.S. Patent Nos. 5,510,121/5,510,418; 5,656,598; 5,686,425; and 5,368,858; and International Publication Nos. WO 92/14480 and WO 93/08828). Prisell *et al*. (1992) evaluated the use of hyaluronic acid as a slow-release vehicle for certain peptide growth factors given subcutaneously (*Int. J. Pharmaceutics* 85:51-56).

GAGs have been shown to disrupt formation of the IGF-I-IGFBP complex in *in vitro* settings (see, for example, Baxter (1990) *Biochem. J.* 271:773-777; Arai *et al*. (1994) *J. Biol. Chem*. 269:20388-20393; Arai *et al.* (1996) *J. Biol. Chem*. 271:6099-6106). They also reportedly modulate proteolysis of the binding proteins (see Arai *et al*. (1994) *Endocrinology* 135:2358-2363) and reportedly modulate activity of certain growth factors in *in vitro* test systems (see, for example, Moscatelli (1988) *J. Cell Biol*. 107:753-759; and Damon *et al*. (1989) *J. Cell. Physiol*. 138:221-226).

The use of GAGs alone as therapeutic agents in animal models has been described in several reports. See, for example, Di Giulio *et al*. (1996) *Society for Neuroscience* 22:1960; Di Giulio *et al*. (1997) *Society for Neuroscience* 23:894; Vergani *et al*. (1997) *Neuroscience Letters* 228:41-44; and Gorio *et al*. (1998) *Neuroscience* 92:1029-1037.

Clearly, better methods for therapy with IGF-I and variants thereof are needed.

### SUMMARY OF THE INVENTION

According to the present invention, there is provided the use of IGF-I or a variant thereof that has IGF-I biological activity and differs from the amino acid sequence of IGF-1 by 10 amino acids or less in combination with at least one glycosaminoglycan, in the manufacture of a medicament for treating an IGF-I-responsive condition in a mammal selected from neurodegenerative disorders, chronic lung disease, acute or chronic renal disorder, acute or chronic liver failure, hepatic cirrhosis, ischemic injury of the heart, liver, or brain, would healing, and organ rejection after transplantation, wherein concurrent therapy using the combination promotes a desired therapeutic response with respect to the IGF-I responsive condition.

There is also provided a pharmaceutical composition comprising a combination of IGF-I or a variant thereof and at least one glycosaminoglycan, wherein said IGF-I or variant thereof that has IGF-I biological activity and differs from the amino acid sequence of IGF-I by 10 amino acids or less and at least one glycosaminoglycan, wherein said IGF-I or variant thereof and said glycosaminoglycan are present in amounts that in combination promote a desired therapeutic response with respect to an IGF-I responsive condition when administered to a mammal undergoing therapy for said IGF-I responsive condition wherein said IGF-1 responsive condition is selected from neurodegenerative disorders, chronic lung disease, acute or chronic renal disorder, acute or chronic liver failure, hepatic cirrhosis, ischemic injury of the heart, liver, or brain, would healing, and organ rejection after transplantation.

### BRIEF DESCRIPTION OF THE DRAWINGS

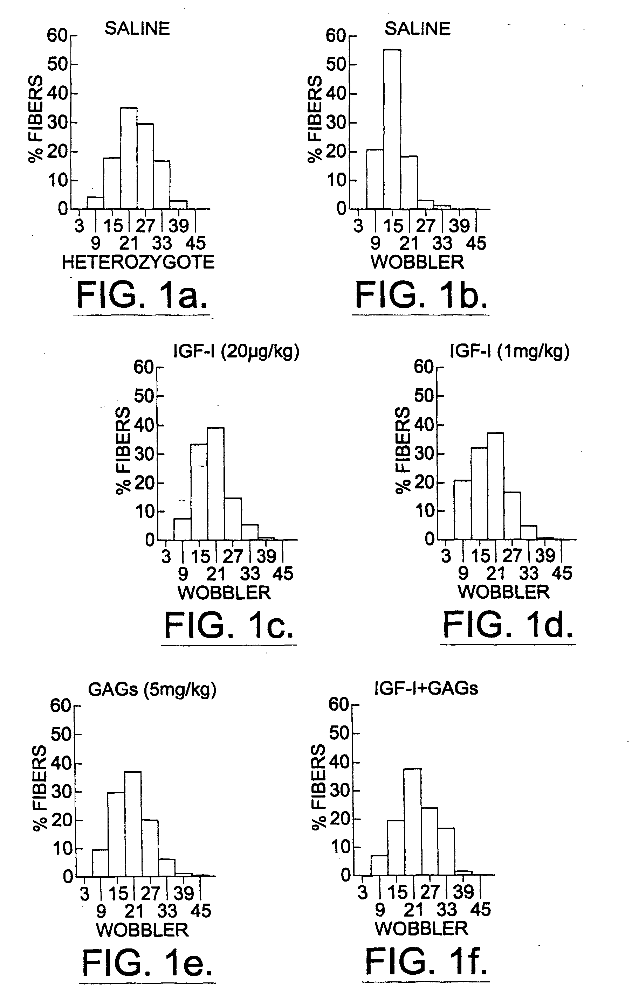

Figure 1 shows the histometric analysis of biceps muscle fiber size expressed as a percentage of the total fiber number measured and present in a certain range of muscle fiber areas of *Wobbler* mice undergoing various drug treatments. Figure 1a represents the control group of heterozygote mice injected daily with saline only. Figures 1b-f represent groups of *Wobbler* mice injected daily with saline only (1b), IGF-I at 20 µg/kg (1c), IGF-I at 1 mg/kg (1d), GAGs at 5 mg/kg (1e), and IGF-I at 20 µg/kg plus GAGs at 1 mg/kg (1f).

### DETAILED DESCRIPTION OF THE INVENTION

Therapy with a combination of IGF-I (or a variant thereof) and at least one GAG causes a physiological response that is beneficial with respect to a condition in the mammal. Therapy with a combination of IGF-I (or a variant thereof) and at least one GAG is desirable for conditions that are responsive to IGF-I, hereinafter referred to as IGF-I responsive conditions. By "IGF-I-responsive condition" is intended conditions that respond in the short term or in the long term either positively or negatively to IGF-I or a variant thereof.

Conditions responsive to IGF-I include chronic lung disease; acute and chronic renal disorders; acute and chronic liver failure; hepatic cirrhosis; ischemic injuries involving the heart, liver, or brain; organ rejection after transplantation; neurodegenerative disorders, such as motor neuron disease, multiple sclerosis, muscular dystrophy, diabetic neuropathy, demyelinating peripheral neurophathies, Parkinson's disease, Alzheimer's disease, and a sequela of traumatic spinal cord lesions. Any such IGF-I responsive condition may benefit from the IGF-I and GAG therapy of the present invention.

By "therapy" is intended treatment of an existing IGF-I responsive condition and preventive or prophylactic procedures performed before the occurrence of an abnormal IGF-I responsive condition. Thus, the mammal receiving therapy may be in a healthy physiological state, or may already have an abnormal IGF-I-responsive condition, or may be prone to having an abnormal IGF-I-responsive condition. Risk factors known to predispose an individual to an abnormal IGF-I-responsive condition can be taken into account when determining whether preventive therapy is desirable. For example, a particular abnormal IGF-I-responsive condition may increase with age, obesity, congenital or developmental defects, metabolic or endocrine disorders, and so forth. Thus, it may be desirable to apply the method of the present invention for preventive purposes depending upon the IGF-I-responsive condition under consideration for treatment.

The present invention may be used with any mammal. Exemplary mammals include, but are not limited to, cats, dogs; horses, cows, sheep, pigs, and more preferably humans.

In accordance with the present invention, IGF-I (or a variant thereof) and GAG are used in combination to promote a desired therapeutic response with respect to an IGF-I-responsive condition. By "desired therapeutic response" is intended an improvement in the condition or in the symptoms associated with the condition. Thus, for example, if the condition undergoing therapy was insufficient muscle mass, a desired therapeutic response would increase in muscle mass. Similarly, if the condition undergoing therapy was a neurological disorder characterized by degeneration of neurons, a desired therapeutic response would be inhibition of degeneration and/or promotion of neuron regeneration. Likewise, if the condition undergoing therapy was a cardiac, liver, or kidney disorder, a desired therapeutic response would be an improvement in cardiac, liver, or kidney function. Thus the desired therapeutic response will depend upon the IGF-I-responsive condition undergoing therapy.

Promotion of a desired therapeutic response with respect to a particular IGF-I-responsive condition in a mammal is achieved via concurrent therapy with both IGF-I (or a variant thereof) at least one GAG. By "concurrent therapy" is intended presentation of IGF-I (or a variant thereof) and at least one GAG to a mammal such that the therapeutic effect of the combination of both substances is caused in the mammal undergoing therapy. Concurrent therapy may be achieved by administering a single pharmaceutical composition containing IGF-I (or a variant thereof) and at least one GAG according to a particular dosing regimen. Alternatively, the IGF-I (or a variant thereof) and at least one GAG may be administered as part of two separate pharmaceutical compositions, one containing IGF-I (or a variant thereof), the other containing at least one GAG. Administration of the separate pharmaceutical compositions can be at the same time or at different times, so long as the therapeutic effect of the combination of both substances is caused in the mammal undergoing therapy. The single or separate pharmaceutical compositions may be administered intravenously, subcutaneously, intramuscularly, intraluminally, intra-articularly, or intraventricularly, or intrapericardially, depending upon the IGF-I-responsive condition undergoing therapy. Alternatively, administration may be achieved with a delivery system as with sustained release from a biodegradable matrix implanted in proximity to a physiological site manifesting the IGF-I condition. This type of administration may be particularly useful in therapy for cartilage or bone disorders where a biodegradable matrix serves as a mold for bone remodeling or cartilage replacement.

Concurrent therapy with an effective amount of the combination of IGF-I (or a variant thereof) and at least one GAG promotes a desired therapeutic response with respect to a particular IGF-I-responsive condition. The respective amounts of IGF-I (or a variant thereof) and at least one GAG that in combination promote the desired therapeutic response are a function of one another. Thus, the amount (or dose) of IGF-I (or a variant thereof) to be used during concurrent therapy is a function of the amount (or dose) of at least one GAG being used in combination with a given dose of IGF-I (or a variant thereof). Likewise, the amount of at least one GAG to be used during concurrent therapy is a function of the amount of IGF-I (or a variant thereof) being used in combination with a given dose of at least one GAG. Administration of at least one GAG concurrently with IGF-I (or a variant thereof) potentiates the effectiveness of IGF-I (or a variant thereof). Thus, inclusion of at least one GAG with IGF-I (or a variant thereof) results in a desired therapeutic response that is improved with respect to that observed with administration of IGF-I (or a variant thereof) alone or at least one GAG alone. Improvement of the desired therapeutic response may be additive in nature or synergistic in nature. Where synergistic, concurrent therapy with IGF-I (or a variant thereof) and at least one GAG results in a desired therapeutic response that is greater than the sum of the desired therapeutic responses achieved with the separate IGF-I (or a variant thereof) and GAG components. Because addition of at least one GAG potentiates the effectiveness of IGF-I (or a variant thereof), a desired therapeutic response that is similar to that achieved with a particular dose of IGF-I (or a variant thereof) alone can be achieved with lower doses of IGF-I (or a variant thereof) administered in combination with at least one GAG. Thus, a dose of IGF-I alone that is not normally therapeutically effective may be therapeutically effective when administered with at least one GAG.

In accordance with the present invention, the amount of at least one GAG used in therapy with IGF-I is an amount that is capable of causing a potential biological effect after administration to a mammal. This is in contrast to amounts of GAG that may be incorporated as carriers, stabilizing agents, chemoattractants, or structural components of pharmaceutical compositions, gel formulations, and biodegradable matrixes. Thus, administration of at least one GAG causes a biological effect that potentiates the desired therapeutic response obtained with IGF-I alone with respect to therapy for the particular IGF-I-responsive condition. According to applicants' invention, the amount of IGF-I (or a variant thereof) when administered in combination with an amount of at least one GAG and the amount of at least one GAG needed to potentiate the effectiveness of a given amount of IGF-I (or a variant thereof) are readily determined by one of ordinary skill in the art without undue experimentation. Factors influencing the mode of administration and the respective amount of IGF-I (or a variant thereof) administered in combination with a given amount of at least one GAG include, but are not limited to, the particular IGF-I-responsive condition undergoing therapy, the severity of the condition, and the age, height, weight, health, and physical condition of the individual undergoing therapy. Generally, a higher dosage is preferred with increasing weight of the mammal undergoing therapy.

The amount of IGF-I (or a variant thereof) is a function of the amount of at least one GAG administered in combination with the IGF-I (or a variant thereof) and vice versa. For example, in one embodiment, the amount of IGF-I (or a variant thereof) ranges from about 1 µg/kg/dose to about 60 µg/kg/dose, preferably from about 2.5 µg/kg/dose to about 45 µg/kg/dose, more preferably from about 5 µg/kg/dose to about 30 µg/kg/dose, while the amount of at least one GAG ranges from about 10 µg/kg/dose to about 15 mg/kg/dose, preferably from about 50 µg/kg/dose to about 10 mg/kg/dose, more preferably from about 0.1 mg/kg/dose to about 5 mg/kg/dose. When the amount of IGF-I (or a variant thereof) ranges from about 5 µg/kg/dose to about 30 µg/kg/dose, the total amount of GAG, which comprises at least one GAG, ranges from about 0.1 mg/kg/dose to about 5 mg/kg/dose. Thus, for example, the amount of IGF-I (or a variant thereof) could be 10, 15, 20, or 25 µg/mg/dose and the total amount of GAG could be 0.1, 0.5, 1, or 2 mg/kg/dose. When the amount of IGF-I (or a variant thereof) is 20 µg/kg/dose, the total amount of GAG is 0.5, 1, or 2 mg/kg/dose, preferably is 1 mg/dose. Generally, the ratio of total GAG to IGF-I (or a variant thereof) on a weight-to-weight basis will be greater than about 10:1, typically will be greater than about 15:1, preferably will be greater than about 20:1, more preferably will be greater than about 30:1, even more preferably will be greater than about 50:1, still more preferably will be greater than about 100:1, even more preferably will be greater than about 500:1, and most preferably will be greater than about 1,000:1. In one embodiment, the ratio of total GAG to IGF-I (or a variant thereof) is about 50:1. As previously noted, the respective amounts of IGF-I (or a variant thereof) and at least one GAG will depend on the IGF-I-responsive condition undergoing concurrent therapy as well as the mode of administration. For example, intrathecal and other local administration of IGF-I (or a variant thereof) typically involves lower doses of IGF-I (or a variant thereof), such as 0.1 to 1 µg/mg/dose.

The term "IGF-I" as used herein refers to insulin-like growth factor I (IGF-I), a single chain peptide having 70 amino acids and a molecular weight of about 7,600 daltons. IGF-I stimulates mitosis and growth processes associated with cell development.

The IGF-I to be administered can be from any animal species including, but not limited to, avian, canine, bovine, porcine, equine, and human. Preferably the IGF-I is from a mammalian species, and more preferably is from a mammal of the same species as the mammal undergoing therapy. The IGF-I may be in the native, recombinantly produced, or chemically synthesized forms as outlined below.

Biologically active variants of IGF-I are also encompassed by the method of the present invention. Such variants should retain IGF-I activities, particularly the ability to bind to IGF-I receptor sites. IGF-I activity may be measured using standard IGF-I bioassays. Representative assays include known radioreceptor assays using placental membranes (see, e.g., U.S. Patent No. 5,324,639; Hall *et al*. (1974) *J. Clin. Endocrinol. and Metab.* 39:973-976; and Marshall *et al*. (1974) *J. Clin. Endocrinol. and Metab.* 39:283-292), a bioassay that measures the ability of the molecule to enhance incorporation of tritiated thymidine, in a dose-dependent manner, into the DNA of BALB/c 3T3 fibroblasts (see, e.g., Tamura *et al*. (1989) *J. Biol. Chem*. 262:5616-5621), and the like; herein incorporated by reference. Preferably, the variant has at least the same activity as the native molecule.

Suitable biologically active variants can be IGF-I fragments, analogues, and derivatives. By "IGF-I fragment" is intended a protein consisting of only a part of the intact IGF-I sequence and structure, and can be a C-terminal deletion or N-terminal deletion of IGF-I. By "analogues" is intended analogues of either IGF-I or an IGF-I fragment that comprise a native IGF-I sequence and structure having one or more amino acid substitutions, insertions, or deletions. Peptides having one or more peptoids (peptide mimics) are also encompassed by the term analogue (see International Publication No. WO 91/04282). By "derivatives" is intended any suitable modification of IGF-I, IGF-I fragments, or their respective analogues, such as glycosylation, phosphorylation, or other addition of foreign moieties, so long as the IGF-I activity is retained. Methods for making IGF-I fragments, analogues, and derivatives are available in the art. See generally U.S. Patent Nos. 4,738,921, 5,158,875, and 5,077,276; International Publication Nos. WO 85/00831, WO 92/04363, WO 87/01038, and WO 89/05822; and European Patent Nos. EP 135094, EP 123228, and EP 128733; herein incorporated by reference.

IGF-I variants will generally have at least 70%, preferably at least 80%, more preferably about 90% to 95% or more, and most preferably about 98% or more amino acid sequence identity to the amino acid sequence of the reference IGF-I molecule. By "sequence identity" is intended the same amino acid residues are found within the IGF-I variant and the reference IGF-I molecule when a specified, contiguous segment of the amino acid sequence of the variant is aligned and compared to the amino acid sequence of the reference molecule. Methods for determining identity between sequences are well known in the art. See, for example, the ALIGN program (Dayhoff (1978) in *Atlas of Protein Sequence and Structure* 5:Suppl. 3 (National Biomedical Research Foundation, Washington, D.C.). For purposes of optimal alignment of the two sequences, the contiguous segment of the amino acid sequence of the variant may have additional amino acid residues or deleted amino acid residues with respect to the amino acid sequence of the reference molecule. The number of amino acid alterations will be 10 or less, preferably 5 or less, more preferably 4 or less, still more preferably 3 or less, even more preferably 2 or less, most preferably 1. The contiguous segment used for comparison to the reference amino acid sequence will comprise at least twenty (20) contiguous nucleotides, and may be 30, 40, 50, 100, or more nucleotides. Corrections for increased sequence identity associated with inclusion of gaps in a variant's amino acid sequence can be made by assigning gap penalties. Methods of sequence alignment are well known in the art.

When considering percentage of amino acid sequence identity, some amino acid residue positions may differ as a result of conservative amino acid substitutions, which do not affect properties of protein function. In these instances, percent sequence identity may be adjusted upwards to account for the similarity in conservatively substituted amino acids. Such adjustments are well known in the art. See, for example, Meyers & Miller (1988) *Computer Applic. Biol. Sci*. 4:11-17.

The art provides substantial guidance regarding the preparation and use of such IGF-I variants, as discussed further below. A fragment of IGF-I will generally include at least 10 contiguous amino acid residues of the full-length molecule, preferably 15 contiguous amino acid residues of the full-length molecule, and most preferably 25 or more contiguous amino acid residues of full-length IGF-I.

Several IGF-I variants are known in the art and include those described in, for example, *Proc. Natl. Acad. Sci. USA* 83 (1986) 4904-4907; *Biochem. Biophys. Res. Commun*. 149 (1987) 398-404; *J. Biol. Chem.* 263 (1988) 6233-6239; *Biochem. Biophys. Res. Commun.* 165 (1989) 766-771; Forsbert *et al*. (1990) *Biochem. J.* 271:357-363; U.S. Patent Nos. 4,876,242 and 5,077,276; and International Publication Nos. WO 87/01038 and WO 89/05822. Representative variants include one with a deletion of Glu-3 of the mature molecule, variants with up to 5 amino acids truncated from the N-terminus, a variant with a truncation of the first 3 N-terminal amino acids (referred to as des(1-3)-IGF-I, des-IGF-I, tIGF-I, or brain IGF), and a variant including the first 17 amino acids of the B chain of human insulin in place of the first 16 amino acids of human IGF-I.

The IGF-I used in the present invention can be in its substantially purified, native, recombinantly produced, or chemically synthesized forms. IGF-I can be isolated and purified from serum or plasma (see Phillips (1980) *New Eng. J Med*. 302:371-380, and European Patent No. EP 123,228). IGF-I can also be chemically synthesized by the solid phase method (see Li *et al*. (1983) *Proc. Natl. Acad. Sci. USA* 80:2216-2220). These references are herein incorporated by reference.

Genetic engineering by recombinant DNA techniques can be the most efficient way of producing IGF-I. The human DNA sequence encoding IGF-I is known and can be introduced into host cells for expression. IGF-I can be produced by recombinant DNA techniques in *E. coli,* yeast, insect, and mammalian cells. Secreted IGF-I can be made by adding a signal sequence to the DNA sequence encoding IGF-I. In addition, the DNA sequence encoding IGF-I can be manipulated to make IGF-I fragments, analogues, or derivatives. Such recombinant DNA techniques are generally available in the art. See, for example, International Publication No. WO 96/07424, where recombinant human IGF-I protein is produced in yeast.

The term "glycosaminoglycan (GAG)" as used herein refers to a group of complex heteropolysaccharides made up primarily of repeating units of disaccharides, in which one sugar is a hexosamine and the other is a uronic acid. Glycosaminoglycans are generally grouped into six distinct classes, including hyaluronate, chondroitin sulfates, dermatan sulfate, keratan sulfate, heparin, and heparan sulfate.

Repeating disaccharide units of 1,4-linked N-acetylglucoasmine and glucuronic acid make up the basic structure of hyaluronate, where the number of repeating units ranges from about 1 to about 5,000. Chondroitin sulfates, which include chondroitin sulfate A and C, consist of repeating disaccharide units of N-acetylgalactosamine and glucuronic acid, with the number of repeating units ranging from about 10 to about 300 for chondroitin sulfate A and from about 20 to about 200 for chondroitin sulfate C. Chondroitin sulfate A is sulfated in the 4 position of N-acetylgalactosamine, while chondroitin sulfate C is sulfated in the 6 position of N-acetylgalactosamine. Dermatan sulfate (also known as chondroitin sulfate B) differs from chondroitin sulfate A in having L-iduronic acid as its predominant uronic acid, although D-glucuronic acid may be present in variable amounts; the number of repeating disaccharide units ranges from about 10 to about 300. Keratan sulfate primarily consists of the repeating disaccharide unit of N-acetylglucosamine and galactose, having no uronic acid in the molecule. Sulfate content varies, with ester sulfate present on C-6 of both galactose and hexosamine; the number of repeating units ranges from about 10 to about 100.

Heparin is formed from repeating units of sulfated glycocyamine and sulfated D-glucuronic acid or L-iduronic acid. Unlike the previous classes of GAGs, heparin contains a-glycosidic linkages. Most glycocyamine residues contain sulfamide linkages, and a small number of glycocyamine residues are N-acetylated. Heparin generally has a sulfate content approaching 2.5 sulfate residues per disaccharide unit. In addition to N-sulfate on C-3 and O-sulfate on C-6 of glycocyamine, heparin may be sulfated on C-3 of glycocyamine and C-2 of the uronic acid. The number of repeating disaccharide units ranges form about 2 to about 3,000. Heparan sulfate differs from heparin in having more N-acetyl groups, fewer N-sulfate groups, and fewer O-sulfate groups.

The highly charged polyanionic nature and macromolecular structure of GAGs are beneficial to their biological roles as lubricants and support elements in connective tissues. They serve as an anchor for cell-specific growth factors and enzymes contained in the extracellular matrix and at the cell surface. Additionally, they play a role in cell adhesion, migration, proliferation, protein secretion, and gene expression.

The amount of GAG to be used in concurrent therapy with IGF-I can be from any one of these six classes, preferably is dermatan sulfate, chondroitin sulfate A, chondroitin sulfate C, heparin, or heparan sulphate, more preferably is heparin having a molecular weight ranging from 35,00 to 45,000 D. Alternatively, the amount of GAG may comprise a mixture of at least two GAGs selected from the six classes. In general, the GAGs include the naturally occurring and synthetic forms. They may be extracted from a natural source and purified and derivatized. Alternatively, they may be synthetically produced or synthesized by modified microorganisms such as bacteria. The GAGs used in the present invention will be pharmaceutical grade, that is they will be substantially free from any toxic compounds or substances.

The IGF-I and GAG as previously described are used in concurrent therapy with the mammal undergoing therapy for the IGF-I-responsive condition. Concurrent therapy may be achieved by administering a single pharmaceutical composition comprising both IGF-I and GAG, or by administering two separate pharmaceutical compositions, one comprising IGF-I and the other comprising at least one GAG.

The pharmaceutical composition comprising both IGF-I and GAG may contain other components that modulate the IGF-I and GAG therapy. Such components include any of the IGF-I binding proteins, IGF-I receptors, and the acid-labile subunit of the IGF-I binding complex. At present, at least six of these, designated IGFBP-1 through IGFBP-6, have been isolated (see, for example, Holly and Martin (1994) *Growth Regul.* 4(Suppl. 1):20-30; Langford *et al*. (1993) *Eur. J. Clin. Invest*. 23(9):503-16). IGFBPs are thought to modulate access of IGF-I to its receptor and hence interfere with IGF-I related responses. The use of ligand inhibitors, specifically analogues of IGF-I, increased the level of free, biologically active IGF-I as measured in *in vitro* and *ex vivo* explant studies (see International Publication No. WO 97/39032). IGFBP-3 may enhance the stimulatory effect of IGF-I on proteoglycan synthesis (see Chevalier *et al*. (1996) *British J. Rheumat.* 35:515-522). In addition, an acid labile glycoprotein also has been shown to be associated with the protein complex formed by IGF-I and its binding proteins. Thus, the therapeutically effective pharmaceutical composition may contain such acid-labile glycoprotein and IGF-I binding proteins, when proven to facilitate the desired therapeutic effect on the IGF-I-responsive condition undergoing therapy. The amount of IGFBPs to be administered with IGF-I can be determined according to the molar ratio between IGF-I and IGFBPs. This molar ratio can range from about 0.5:1 to about 3:1, preferably about 1:1 (see U.S. Patent No. 5,187,151). Alternatively, the pharmaceutical composition may include agents that disrupt IGF-I binding to IGFBPs and which may be effective in enhancing IGF-I and GAG therapy.

In addition to these components, the pharmaceutical composition comprising IGF-I and GAG may include one or more protease inhibitors. An exemplary protease inhibitor is sodium pentosan polysulfate (PPS), a polysulfated polysaccharide. This protease inhibitor has efficacy in treating osteoarthritis in combination with low dosages of IGF-I (1 µg IGF-I intra-articularly 3 times per week) (Rogachefsky *et al*. (1993) *Osteoarthritis and* *Cartilage* 1:105-114). Such a protease inhibitor can be administered by other routes, such as intramuscularly, during concurrent administration of the effective does of IGF-I and GAG.

The pharmaceutical composition in accordance with the present invention may further comprise one or more other therapeutic agents that are effective in treating other conditions in the individual, as long as the biochemical actions of the additional therapeutic agents do not interfere with the efficacy of intended action of the IGF-I and GAG therapy. Examples of such agents include, but are not limited to, antibiotics, anti-inflammatory agents, and the like.

A pharmaceutically acceptable carrier should be mixed with the IGF-I, GAG, and other components in the pharmaceutical composition. By "pharmaceutically acceptable carrier" is intended a carrier that is conventionally used in the art to facilitate the storage, administration, and/or the healing effect of the therapeutic ingredients. A carrier may also reduce any undesirable side effects of the IGF-I. A suitable carrier should be stable, i.e., incapable of reacting with other ingredients in the formulation. It should not produce significant local or systemic adverse effect in recipients at the dosages and concentrations employed for therapy. Such carriers are generally known in the art. Suitable carriers for this invention are those conventionally used large stable macromolecules such as albumin, gelatin, collagen, polysaccharide, monosaccarides, polyvinylpyrrolidone, polylactic acid, polyglycolic acid, polymeric amino acids, fixed oils, ethyl oleate, liposomes, glucose, sucrose, lactose, mannose, dextrose, dextran, cellulose, mannitol, sorbitol, polyethylene glycol (PEG), and the like. Slow-release carriers, such as hyaluronic acid, may also be suitable. See particularly Prisell *et al*. (1992) *Int. J. Pharmaceu*. 85:51-56 and U.S. Patent No. 5,166,331. Inclusion of hyaluronic acid and other polymers may have an additional beneficial effect on the IGF-I-responsive disorder osteoarthritis. See particularly Bragantini (1987) *Clin. Trials J*. 24(4):333-340; Dougados *et al*. (1993) *Osteoarthritis and Cartilage 1:97-103;* and Lussier *et al*. (1996) *J. Rheum*. 23:1579-1585; herein incorporated by reference. Other acceptable components in the composition include, but are not limited to, buffers that enhance isotonicity such as water, saline, phosphate, citrate, succinate, acetic acid, and other organic acids or their salts.

Preferred pharmaceutical compositions may incorporate buffers having reduced local pain and irritation resulting from injection of IGF-I compositions. Such buffers include, but are not limited to, low phosphate buffers and succinate buffers. For example, International Publication No. WO 94/15584 describes isotonic IGF-I solution at pH 5.5 to 6.5 with phosphate buffer present in an amount less than 50 mmol/L, which are reported to result in reduced pain upon injection. The pharmaceutical composition may additionally comprise a solubilizing compound that is capable of enhancing the solubility of IGF-I or an IGF-I variant.

For the purposes of this invention, the pharmaceutical composition comprising IGF-I and GAG should be formulated in a unit dosage and in an injectable or infusible form such as solution, suspension, or emulsion. It can also be in the form of lyophilized powder, which can be converted into solution, suspension, or emulsion before administration. The pharmaceutical composition having IGF-I and GAG is preferably sterilized by membrane filtration and is stored in unit-dose or multi-dose containers such as sealed vials or ampules.

The method for formulating a pharmaceutical composition is generally known in the art. A thorough discussion of formulation and selection of pharmaceutically acceptable carriers, stabilizers, and isomolytes can be found in *Remington's Pharmaceutical Sciences* (18^{th} ed.; Mack Pub. Co.: Eaton, Pennsylvania, 1990), herein incorporated by reference.

The IGF-I of the present invention can also be formulated in a sustained-release form to prolong the presence of the pharmaceutically active IGF-I in the treated mammal, generally for longer than one day. The therapeutically effective dose of GAG may be incorporated into this sustained-release formulation or administered as part of a separate pharmaceutical composition. Many methods of preparation of a sustained-release formulation are known in the art and are disclosed in *Remington's Pharmaceutical Sciences* (18^{th} ed.; Mack Pub. Co.: Eaton, Pennsylvania, 1990), herein incorporated by reference. Generally, the IGF-I can be entrapped in semipermeable matrices of solid hydrophobic polymers. The matrices can he shaped into films or microcapsules. Examples of such matrices include, but are not limited to, polyesters, copolymers of L-glutamic acid and gamma ethyl-L-glutamate (Sidman *et al*. (1983) *Biopolymers* 22:547-556), poly-actides (U.S. Patent No. 3,773,919 and EP 58,481), polyactate polyglycolate (PLGA), hydrogels (see, for example, Langer *et al*. (1981) *J. Biomed. Mater. Res.* 15:167-277; Langer (1982) *Chem. Tech.* 12:98-105), non-degradable ethylene-vinyl acetate, degradable lactic acid-glycolic acid copolymers such as the Lupron Depot™, and poly-D-(-)-3-hydroxybutyric acid (EP 133,988). Suitable microcapsules can also include hydroxymethylcellulose or gelatin-microcapsules and poly-methylmethacylate microcapsules prepared by coacervation techniques or by interfacial polymerization. In addition, microemulsions or colloidal drug delivery systems such as liposomes and albumin microspheres, may also be used. See *Remington's Pharmaceutical Sciences* (18^{th} ed.; Mack Pub. Co.: Eaton, Pennsylvania, 1990). One such sustained-release formulation is Depo IGF-I (Depofoam), wherein recombinant human IGF-I is encapsulated in multivesicular liposomes, as disclosed in the copending application entitled *"High and Low Load Formulations of IGF-I in Multivesicular Liposomes*," U.S. Patent Application Serial No. 08/925531, filed September 8, 1997, herein incorporated by reference. The mean residence time of IGF-I in the joint is approximately two-fold longer with Depo IGF-I than with free IGF-I (8.4 hours versus 4.1 hours, respectively). By "residence time" is intended the amount of time during which the concentration of IGF-I within the mammal undergoing treatment for an IGF-I-responsive condition remains high enough above baseline to be therapeutically effective.

In one embodiment of the invention, concurrent therapy with IGF-I and at least one GAG is achieved intermittently. By "intermittent concurrent therapy" is intended a period of concurrent therapy with IGF-I and at least one GAG, followed by a time period of discontinuance, which is then followed by another period of concurrent therapy with IGF-I and at least one GAG, and so forth. Concurrent therapy, where IGF-I and GAG are presented in combination to a mammal, may be achieved in a continuous manner, as for example with a sustained-release formulation, or it may be achieved according to a desired daily dosage regimen, as for example with one two, or three or more injections per day of a single pharmaceutical composition containing both IGF-I and at least one GAG, or of two separate pharmaceutical compositions, one containing IGF-I, the other containing at least one GAG. Regardless of the mode of administration, IGF-I and at least one GAG are administered at their respective amounts that in combination promote a desired therapeutic response with respect to a particular IGF-I-responsive condition. By "time period of discontinuance" is intended a discontinuing of the continuous sustained-release or daily administration of IGF-I in combination with at least one GAG. The time period of discontinuance may be longer or shorter than the period of continuous sustained-release or daily administration. During the time period of discontinuance, the IGF-I and GAG levels within the mammal undergoing therapy for an IGF-I-responsive condition are substantially below the maximum levels obtained during therapy. The preferred length of the discontinuance period depends on the amounts of IGF-I and total GAG that are used in combination, the form of IGF-I used, and the type of administration used. For example, when a sustained-release formulation is used, the discontinuance period must be extended to account for the greater residence time of IGF-I within the mammal undergoing therapy. Alternatively, the frequency of administration of the effective amount of the sustained-release formulation containing both IGF-I and at least one GAG can be decreased accordingly.

For example, concurrent therapy with IGF-I and at least one GAG might follow an intermittent schedule where an effective amount of the combination of IGF-I and at least one GAG is administered according to a daily dosage regimen, such as once a day, twice a day, or three or more times a day, with the daily dosage regimen occurring every day, every other day, three times a week, twice a week, once a week, every other week, once a month, once every two months, once every three months, twice a year, and so forth. An intermittent schedule of concurrent therapy with IGF-I and GAG may continue in a mammal undergoing therapy until the desired therapeutic response with respect to a particular IGF-I-responsive condition is achieved.

In another embodiment of the present invention, intermittent concurrent therapy with IGF-I and at least one GAG is cyclic. By "cyclic" is intended intermittent concurrent therapy accompanied by breaks in the concurrent therapy, with cycles ranging from about 1 month to about 2, 3, 4, 5, or 6 months, more preferably about 3 months to about 6 months. For example, the concurrent therapy schedule might be intermittent concurrent therapy with IGF-I and at least one GAG, wherein an effective amount of the combination of IGF-I and at least one GAG is administered once per week for 4 weeks, followed by a break in intermittent concurrent therapy for a period of 3 months, followed by intermittent concurrent therapy with IGF-I and at least one GAG, wherein an effective amount of the combination of IGF-I and at least one GAG is administered once per week for 4 weeks, followed by a break in intermittent concurrent therapy for a period of 3 months, and so forth. As another example, an effective amount of the combination of the two substances may be administered once per week for 2 weeks, followed by a break in intermittent concurrent therapy for a period of 1 month, followed by administration of an effective amount of the combination of the two substances once per week for 2 weeks, followed by a break in intermittent concurrent therapy for a period of 1 month, and so forth. A cyclic intermittent schedule of concurrent therapy with IGF-I and at least one GAG may continue until the desired therapeutic response with respect to a particular IGF-I response condition is achieved. Intermittent therapy with IGF-I is referred to in U.S. Patent No. 5,741,776.

Alternatively, concurrent therapy with IGF-I and at least one GAG may be achieved directly at the site with a sustained-release device or delivery system. Such devices are well known in the art (see, for example, U.S. Patent No. 5206023). For example, a biodegradable matrix comprising an effective amount of the combination of IGF-I and at least one GAG in a sustained-release form may be implanted within a mammal. Such a device would allow for sustained-release of an effective amount of the combination of both substances. As the matrix degrades, the effective amount of the combination of IGF-I and at least one GAG promote a desired therapeutic response with respect to the particular IGF-I-responsive condition.

It should be apparent to a person skilled in the art that variations may be acceptable with respect to the effective amount of the combination of IGF-I and at least one GAG and the frequency of the administration of this effective amount in this embodiment of the invention. Some minor degree of experimentation may be required to determine the relative amounts of IGF-I and GAG to be used in concurrent therapy this being well within the capability of one skilled in the art once apprised of the present disclosure.

Thus, promotion of a desired therapeutic response with respect to a particular IGF-I-responsive condition undergoing therapy may be achieved via concurrent therapy with IGF-I and at least one GAG. In addition, methods for manipulating the level of naturally produced IGF-I in combination with concurrent therapy with IGF-I and GAG are also encompassed by the present invention. Thus, the level of naturally produced IGF-I could be genetically manipulated in addition to concurrently administering at least one GAG alone or in combination with IGF-I.

The interest in gene therapy as a means of treating inherited or acquired diseases has led to the development of methods for transferring genetic information, more particularly for delivering nucleotide sequences encoding human genes using viral-mediated gene transfer systems. Such viral-mediated gene transfer systems enable delivery of desired genetic information, in this case a nucleotide sequence encoding IGF-I, to a selected cell or tissue and its subsequent expression there under the direction of the viral promoter. Viral-mediated gene transfer systems are known in the art. See, for example, U.S. Patent Nos. 5,707,618; 5,714,353; and 5,672,344. In this manner, increases in the amount of IGF-I can be partially achieved *in vivo* by increasing production of IGF-I.

Efficacy of a particular amount of the combination of IGF-I and at least one GAG, and the best mode of administration, may be measured in accordance with its ability to promote the desired therapeutic response with respect to the particular IGF-I-responsive condition undergoing therapy.

The following examples are offered by way of illustration and not by way of limitation.

### EXPERIMENTAL

The following examples illustrate the efficacy of using both IGF-I and at least one glycosaminoglycan as therapy for a neurodegenerative disorder.

### Example 1

### Methods

This pharmacological research was performed using *Wobbler* mice, a genetic animal model of ALS (amyotrophic lateral sclerosis) characterized by early loss of motor neurons and reduced forelimb muscle function.

Heterozygous *Wobbler* mice were bred under standard housing conditions (22 ± 2°C, 65% humidity, artificial lights from 06.00-20.00 h). A standard dry diet and water were available *ad libitum* throughout the experiment. All experimental protocols were approved by the Review Committee of the Department of Pharmacology, and met the Italian guidelines for laboratory animals, which conform with the European Communities Directive of November 1986 (86/609/EEC).

Upon clear diagnosis at three weeks of age, homozygous animals were randomly assigned to five treatment groups that received, respectively, the following daily doses by means of subcutaneous injections:
-- Vehicle (saline)
-- rhIGF-I (20 µg/kg)
-- rhIGF-I (1 mg/kg)
-- GAGs (1 mg/kg)
-- rhIGF-I (20 µg/kg) + GAGs (1 mg/kg)

The human IGF-I was recombinantly produced in *E. coli* and obtained from a distributor (INALCO (Milano, Italy), a commercial representative of PeproTech, Inc. (Princeton Business Park, Rock Hill, New Jersey), Catalog No. 100-11) for research purposes only. GAGs constituted the following mixture:
-- Slow moving heparin 19.6%
-- Fast moving heparin 44.9%
-- Dermatan Sulfate 28.8%
-- Condroitin sulfate A and C 6.7%

### Results and Discussion

Table 1 shows the number of triceps motor neurons surviving in *Wobbler* mice at 9 weeks of age as determined by retrograde labeling of horseradish peroxidase as previously described by Baulac *et al*. (1983) *Neurosci. Letters* 37:99-104. The number of triceps motor neurons at 3 weeks of age was 160-180.

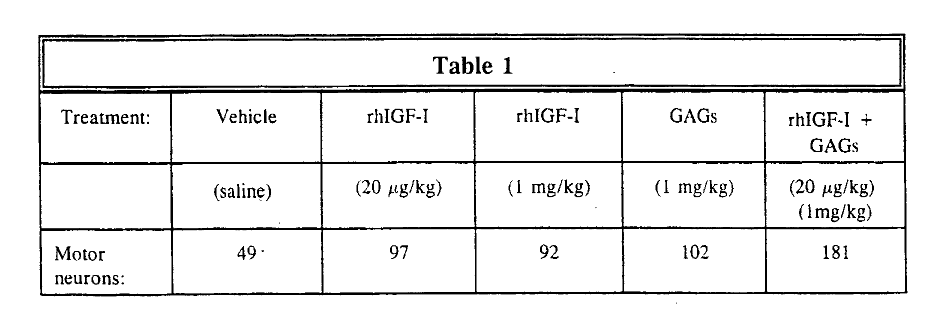

Table 2 shows mice body weight (grams) at 9 weeks of age and the average weekly body weight growth (Δ/week).

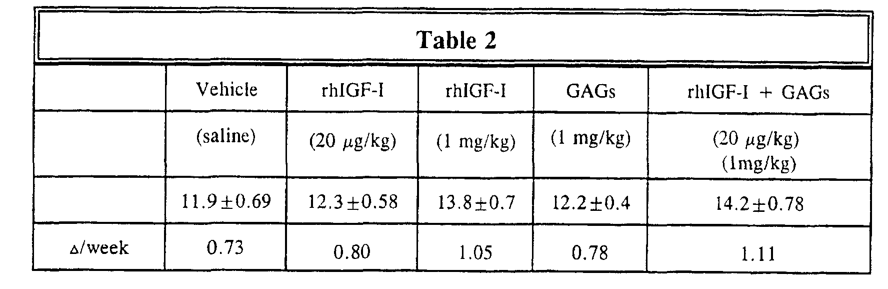

Table 3 shows *Wobbler* mice grip strength (g) determined as described by Mitsumoto *et al*. (1994) *Ann. Neurol.* 36:142-148.

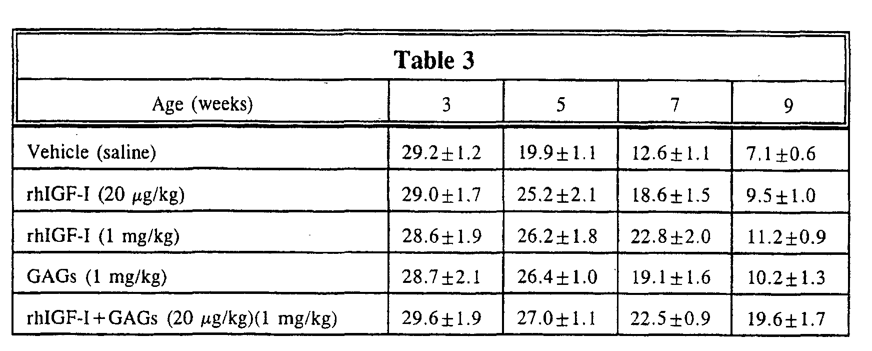

Table 4 shows *wobbler* mice holding time (sec) as assessed with the method described by Mitsumoto *et al*. (1994) *Ann. Neurol*. 36:142-148.

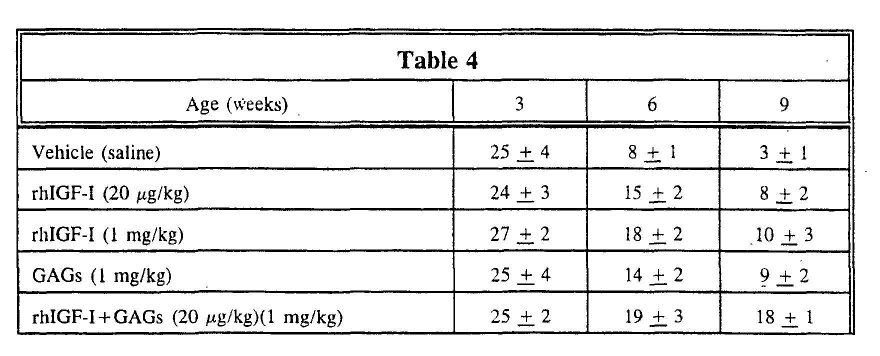

Table 5 describes the mean time (sec) occurring to run along a 10-cm stretch as determined by Mitsumoto *et al*. (1994) *Ann. Neurol*. 36:142-148.

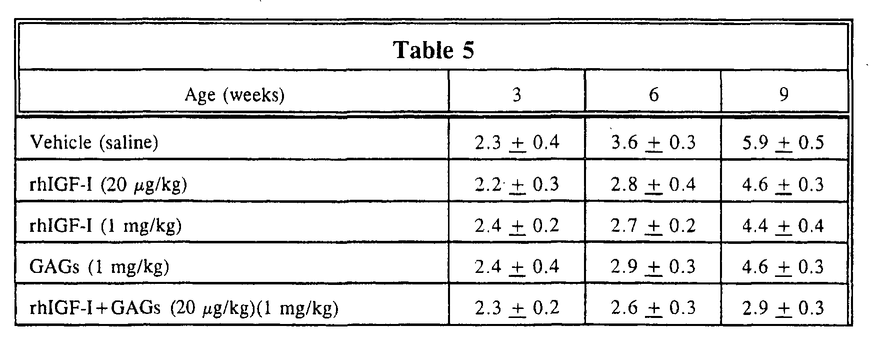

In addition to the data reported in Tables 1-5, Figure 1 shows the histometric analysis of biceps muscle fiber size expressed as % of the total fiber number measured and present in a certain range of muscle fiber areas. For comparison, the biceps fiber size distribution of the heterozygotes that do not develop the disease is also shown. The rhIGF-I + GAGs treatment prevented biceps muscle fiber atrophy so as to give a size distribution similar to biceps muscle from heterozygotes.

All the parameters that we have investigated show that the pharmaceutical composition made up of rhIGF-I and GAGs is surprisingly effective in promoting desired therapeutic treatment effects in the animal model of ALS and SMA (spinal muscular atrophy). In particular, it is remarkable that the treatment of *Wobbler* mice with the composition in accordance with the present invention allows the blockade of motor neuron death as shown clearly by the data of Table 1. Such an effect cannot be obtained by single administration of rhIGF-I alone or of GAGs alone. In addition, these compositions allows for treatment with lower doses of rhIGF-I, thus avoiding possible undesirable side effects.

### Example 2

### Methods

Motor neuron disease (MND) is an autosomal dominant neurological disease that occurs in the C57b1/6 mice of both sexes (Messer *et* *al*. (1992) *Genomics* 18:797-802). These mice develop motor abnormalities initially in the hindlimbs, then animals show greatly reduced spontaneous movement and die before 1 year of age. Because animals exhibit reduced mobility, the disease has been proposed as a model of ALS (Messer *et al*. (1993) *Neuromusc. Disorder* 3:129-134). The number of motor neurons surviving and the isometric tension developed by the peroneal muscles after 10 months of life were evaluated using the fine treatment groups described in Example 1 and at least 6 mice per experimental group. Daily doses of the following were injected subcutaneously from 120 days of life up to sacrifice: rhIGF-I at 20 µg/kg or 1 mg/kg; GAGs at 1 mg/kg; and GAGs at 1 mg/kg + rhIGF-I at 20 µg/kg. Isometric tension was evaluated *in vitro* using the sciatic nerve-peroneal muscle dissected preparation, as previously described (Gorio *et al*. (1997) *Eur. J. Neurosci*. 9:1748-1753).

### Results and Discussion

In the MND mice, there was only a small, not significant loss of motor neurons, and no effect of the drug treatments was observed.

However, the effect of drug treatment was marked for isometric tension. Following the stimulation at 1 hertz or at 50 hertz of the sciatic nerve, the peroneal muscle tension was as shown in Table 6:

**Table 6**

| | 1 hz | 50 hz |
|---|---|---|
| Vehicle (saline) | 3.1 ± 0.1 | 2.1 ± 0.1 |
| rhICF-1 (20 µg/kg) | 4.2 ± 0.2* | 3.6 ± 0.1** |
| rhIGF-I (1 mg/kg) | 4.0 ± 0.3* | 3.6 ± 0.3** |
| GAGs (1 mg/kg) | 4.3 ± 0.1* | 3.8 ± 0.2** |
| rhIGF-I + GAGs (20 µg/kg) (1mg/kg) | 4.6 ± 0.2* | 4.8±0.2**§ |

| | | |
|---|---|---|
| * Indicates the difference vs. saline; § vs. single treatment. Data were analyzed with Anova followed by Dunnett's t test. | | |

These data suggest a greater strength of the peroneal nerve-muscle preparation of MND mice after any of the treatments used. However, the high frequency nerve stimulation (50 Hertz), which normally causes a greater (tetanic) tension in muscles of normal animals, caused a loss of strength in the untreated MND animals (vehicle group). In other words, muscles of MND mice fatigue immediately. Such a decrease was significantly smaller in single treated (rhIGF-I or GAGs) MND mice, but was fully prevented by the co-treatment with rhIGF-I and GAGs. These data suggest that co-treatment prevents the decay of neuromuscular function in MND mice. This conclusion is dictated by the lack of motor neuron loss in this disease.

Although the foregoing invention has been described in some detail by way of illustration and example for purposes of clarity of understanding, it will be apparent that certain changes and modifications may be practiced within the scope of the appended claims.

All publications and patent applications mentioned in the specification are indicative of the level of those skilled in the art to which this invention pertains.

## Claims

1. Use of IGF-I or a variant thereof that has IGF-I biological activity and differs from the amino acid sequence of IGF-I by 10 amino acids or less in combination with at least one glycosaminoglycan, in the manufacture of a medicament for treating an IGF-I-responsive condition in a mammal selected from neurodegenerative disorders, chronic lung disease, acute or chronic renal disorder, acute or chronic liver failure, hepatic cirrhosis, ischemic injury of the heart, liver, or brain, would healing, and organ rejection after transplantation, wherein concurrent therapy using the combination promotes a desired therapeutic response with respect to the IGF-I responsive condition.

2. Use according to claim 1, wherein said mammal is a human and wherein human IGF-I is used in the manufacture of said medicament.

3. Use according to claim 1 or 2, wherein said desired therapeutic response is greater than a response that would be observed with said IGF-I or variant thereof alone or said glycosaminoglycan alone.

4. Use according to claim 1, 2 or 3 wherein said concurrent therapy comprises administering to said mammal said IGF-I or variant thereof and said glycosaminoglycan together in a single pharmaceutical composition.

5. Use according to claim 1, 2 or 3 wherein said concurrent therapy comprises administering to said mammal said IGF-I or variant thereof and said glycosaminoglycan in two separate pharmaceutical compositions, wherein one of said compositions comprises said IGF-I or variant thereof and wherein the other of said compositions comprises said glycosaminoglycan.

6. Use according to claim 4 or 5, wherein said glycosaminoglycan is 35,000-45,000 D heparin, dermatan sulfate, chondroitin sulfate A, chondroitin sulfate C, or heparan sulfate.

7. Use according to any one of the preceding claims wherein said IGF-I-responsive condition is a neurodegenerative disorder.

8. Use according to claim 7, wherein said neurodegenerative disorder is motor neuron disease, multiple sclerosis, muscular dystrophy, diabetic neuropathy, demyelinating peripheral neuropathies, Parkinson's disease, Alzheimer's disease, or a sequela of traumatic spinal cord lesions.

9. Use according to claim 8, wherein the amount of IGF-I or variant thereof administered is from about 5 µg/kg/dose to about 30 µg/kg/dose and the amount of glycosaminoglycan administered is from about 0.1 mg/kg/dose to about 5 mg/kg/dose.

10. Use according to claim 9, wherein the ratio of glycosaminoglycan to IGF-I or variant thereof is about 50:1.

11. A pharmaceutical composition comprising IGF-I or a variant thereof that has IGF-I biological activity and differs from the amino acid sequence of IGF-I by 10 amino acids or less and at least one glycosaminoglycan, wherein said IGF-I or variant thereof and said glycosaminoglycan are present in amounts that in combination promote a desired therapeutic response with respect to an IGF-I responsive condition when administered to a mammal undergoing therapy for said IGF-I responsive condition wherein said IGF-I responsive condition is selected from neurodegenerative disorders, chronic lung disease, acute or chronic renal disorder, acute or chronic liver failure, hepatic cirrhosis, ischemic injury of the heart, liver, or brain, would healing, and organ rejection after transplantation.

12. A composition according to claim 11, wherein said IGF-I or variant thereof is at a concentration of about 15 µg/ml to about 2,000 µg/ml and said glycosaminoglycan is at a concentration of about 0.3 mg/ml to about 350 mg/ml.

13. A composition according to claim 11 or 12, wherein the IGF-I is human IGF-I.

## Patentansprüche

1. Verwendung von IGF-I oder einer Variante davon, die biologische IGF-I-Aktivität hat und sich von der Aminosäuresequenz von IGF-I um 10 Aminosäuren oder weniger unterscheidet, in Kombination mit wenigstens einem Glycosaminoglycan bei der Herstellung eines Medikaments zur Behandlung eines auf IGF-I ansprechenden Zustands bei einem Säuger, ausgewählt aus neurodegenerativen Störungen, chronischer Lungenkrankheit, akuter oder chronischer Nierenerkrankung, akutem oder chronischem Leberversagen, Leberzirrhose, ischämischer Verletzung von Herz, Leber oder Gehirn, Wundheilung und Organabstoßung nach Transplantation, wobei eine gleichzeitige Therapie unter Verwendung der Kombination eine gewünschte therapeutische Reaktion bezüglich des auf IGF-I ansprechenden Zustands begünstigt.

2. Verwendung nach Anspruch 1, wobei der Säuger ein Mensch ist und wobei humaner IGF-I bei der Herstellung des Medikaments eingesetzt wird.

3. Verwendung nach Anspruch 1 oder 2, wobei die gewünschte therapeutische Reaktion stärker ist als eine Reaktion, die mit dem IGF-I oder einer Variante davon allein oder dem Glycosaminoglycan allein beobachtet würde.

4. Verwendung nach Anspruch 1, 2 oder 3, wobei die gleichzeitige Therapie eine Verabreichung des IGF-I oder einer Variante davon und des Glycosaminoglycans zusammen in einer einzigen pharmazeutischen Zusammensetzung an den Säuger umfasst.

5. Verwendung nach Anspruch 1, 2 oder 3, wobei die gleichzeitige Therapie Verabreichung des IGF-I oder einer Variante davon und des Glycosaminoglycans in zwei getrennten pharmazeutischen Zusammensetzungen an den Säuger umfasst, wobei eine der Zusammensetzungen den IGF-I oder eine Variante davon umfasst, und wobei die andere der Zusammensetzungen das Glycosaminoglycan umfasst.

6. Verwendung nach Anspruch 4 oder 5, wobei das Glycosaminoglycan Heparin mit 35 000 bis 45 000 D, Dermatansulfat, Chondroitinsulfat A, Chondroitinsulfat C oder Heparansulfat ist.

7. Verwendung nach einem der vorangehenden Ansprüche, wobei der auf IGF-I ansprechende Zustand eine neurodegenerative Störung ist.

8. Verwendung nach Anspruch 7, wobei die neurodegenerative Störung eine Motoneuronen-Krankheit, Multiple Sklerose, Muskeldystrophie, diabetische Neuropathie, demyelinisierende periphere Neuropathien, Parkinsonerkrankung, Alzheimererkrankung oder eine Folge traumatischer Rückenmarksläsionen ist.

9. Verwendung nach Anspruch 8, wobei die Menge an IGF-I oder einer Variante davon, die verabreicht wird, etwa 5 µg/kg/Dosis bis etwa 30 µg/kg/Dosis ist und die verabreichte Menge an Glycosaminoglycan etwa 0,1 mg/kg/Dosis bis etwa 5 mg/kg/Dosis ist.

10. Verwendung nach Anspruch 9, wobei das Verhältnis von Glycosaminoglycan zu IGF-I oder einer Variante davon etwa 50:1 ist.

11. Pharmazeutische Zusammensetzung, die IGF-I oder eine Variante davon, die biologische IGF-I-Aktivität hat und sich von der Aminosäuresequenz von IGF-I um 10 Aminosäuren oder weniger unterscheidet, und wenigstens ein Glycosaminoglycan umfasst, wobei der IGF-I oder die Variante davon und das Glycosaminoglycan in Mengen vorliegen, die in Kombination eine gewünschte therapeutische Reaktion bezüglich eines auf IGF-I ansprechenden Zustandes begünstigen, wenn diese einem Säuger verabreicht wird, der eine Therapie für diesen auf IGF-I ansprechenden Zustand durchmacht, wobei der auf IGF-I ansprechende Zustand aus neurodegenerativen Störungen, chronischer Lungenkrankheit, akuter oder chronischer Nierenstörung, akutem oder chronischem Leberversagen, Leberzirrhose, ischämischer Verletzung von Herz, Leber oder Gehirn, Wundheilung und Organabstoßung nach Transplantation ausgewählt ist.

12. Zusammensetzung nach Anspruch 11, wobei der IGF-I oder eine Variante davon in einer Konzentration von etwa 15 µg/l bis etwa 2 000 µg/ml vorliegt und das Glycosaminoglycan in einer Konzentration von etwa 0,3 mg/ml bis etwa 350 mg/ml vorliegt.

13. Zusammensetzung nach Anspruch 11 oder 12, wobei der IGF-I humaner IGF-I ist.

## Revendications

1. Utilisation d'IGF-I, ou d'un de ses variants qui a une activité biologique d'IGF-I et diffère de la séquence d'acides aminés d'IGF-I par 10 acides aminés ou moins, en combinaison avec au moins un glycosaminoglycane, dans la fabrication d'un médicament pour traiter un état qui répond à IGF-I chez un mammifère, choisi parmi des troubles neurodégénératifs, une maladie pulmonaire chronique, un trouble rénal aigu ou chronique, une insuffisance hépatique aiguë ou chronique, une cirrhose hépatique, une lésion ischémique du coeur, du foie ou du cerveau, la cicatrisation de plaies, et le rejet d'organe après transplantation, laquelle thérapie simultanée utilisant la combinaison favorise une réponse thérapeutique désirée relative à l'état qui répond à IGF-I.

2. Utilisation selon la revendication 1, dans laquelle ledit mammifère est un humain et dans laquelle on utilise IGF-I humain dans la fabrication dudit médicament.

3. Utilisation selon la revendication 1 ou 2, dans laquelle ladite réponse thérapeutique désirée est supérieure à une réponse que l'on observerait avec ledit IGF-I ou l'un de ses variants seul, ou ledit glycosaminoglycane seul.

4. Utilisation selon la revendication 1, 2 ou 3, dans laquelle ladite thérapie simultanée comprend l'administration audit mammifère dudit IGF-I ou de l'un de ses variants et dudit glycosaminoglycane ensemble dans une seule composition pharmaceutique.

5. Utilisation selon la revendication 1, 2 ou 3, dans laquelle ladite thérapie simultanée comprend l'administration audit mammifère dudit IGF-I ou l'un de ses variants et dudit glycosaminoglycane dans deux compositions pharmaceutiques séparées, où l'un desdites compositions comprend ledit IGF-I ou l'un de ses variants et où l'autre desdites compositions comprend ledit glycosaminoglycane.

6. Utilisation selon la revendication 4 ou 5, dans laquelle ledit glycosaminoglycane est l'héparine 35 000-45 000 D, le dermatan-sulfate, le chondroïtine sulfate A, le chondroïtine sulfate C, ou l'héparan-sulfate.

7. Utilisation selon l'une quelconque des revendications précédentes dans laquelle ledit état qui répond à IGF-I est un trouble neurodégénérératif.

8. Utilisation selon la revendication 7, dans laquelle ledit trouble neurodégénératif est une maladie des neurones moteurs, la sclérose en plaque, une dystrophie musculaire, une neuropathie diabétique, des neuropathies périphériques démyélisantes, la maladie de Parkinson, la maladie d'Alzheimer ou une séquelle de lésions traumatiques de la moelle épinière.

9. Utilisation selon la revendication 8, dans laquelle la quantité administrée d'IGF-I ou de l'un de ses variants est d'environ 5 µg/kg/dose à environ 30 µg/kg/dose et la quantité de glycosaminoglycane administrée est d'environ 0,1 mg/kg/dose à environ 5 mg/kg/dose.

10. Utilisation selon la revendication 9, dans laquelle le rapport de glycosaminoglycane à IGF-I ou un de ses variants est d'environ 50/1.

11. Composition pharmaceutique comprenant IGF-I ou l'un de ses variants qui a une activité biologique d'IGF-I et diffère de la séquence d'acides aminés d'IGF-I par 10 acides aminés ou moins et d'au moins un glycosaminoglycane, dans laquelle ledit IGF-I ou l'un de ses variants et ledit glycosaminoglycane sont présents en quantités qui, en combinaison, favorisent une réponse thérapeutique désirée relativement à un état qui répond à IGF-I lorsqu'on les administre à un mammifère subissant une thérapie pour ledit état qui répond à IGF-I, ledit état qui répond à IGF-I étant choisi parmi les troubles neurodégénératifs, une maladie pulmonaire chronique, un trouble rénal aigu ou chronique, une insuffisance hépatique aiguë ou chronique, une cirrhose hépatique, une lésion ischémique du coeur, du foie ou de cerveau, la cicatrisation de plaies, et le rejet d'organe après transplantation.

12. Composition selon la revendication 11, dans laquelle ledit IGF-I ou l'un de ses variants est à une concentration d'environ 15 µg/ml à environ 2000 µg/ml et ledit glycosaminoglycane est à une concentration d'environ 0,3 mg/ml à environ 350 mg/ml.

13. Composition selon la revendication 11 ou 12, dans laquelle l'IGF-I est l'IGF-I humain.
